(19) Europäisches Patentamt
European Patent Office
Office européen des brevets

(11) EP 4 227 951 A1

(12) **EUROPEAN PATENT APPLICATION**

(43) Date of publication:
**16.08.2023 Bulletin 2023/33**

(21) Application number: **22156423.0**

(22) Date of filing: **11.02.2022**

(51) International Patent Classification (IPC):
***G16C 20/30*** *(2019.01)* ***G16C 20/50*** *(2019.01)*
***G16C 20/70*** *(2019.01)*

(52) Cooperative Patent Classification (CPC):
**G16C 20/30; G16C 20/50; G16C 20/70;** G06N 3/02; G06N 3/126

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Samsung Display Co., Ltd.**
**Gyeonggi-do 17113 (KR)**

(72) Inventors:
- **Ruoff, Patrick**
  **76131 Karlsruhe (DE)**
- **Schmalzl, Markus**
  **76669 Bad Schönborn (DE)**
- **Schniewind, Matthias**
  **76185 Karlsruhe (DE)**

(74) Representative: **Jacobi, Markus Alexander**
**Patentanwälte**
**Isenbruck Bösl Hörschler PartG mbB**
**Eastsite One**
**Seckenheimer Landstrasse 4**
**68163 Mannheim (DE)**

# (54) METHOD FOR PREDICTING AND OPTIMIZING PROPERTIES OF A MOLECULE

(57) A computer-implemented method for predicting a value of a physical and/or chemical property (180a, 180b) of a molecule, said method uses as input a molecular structure of the molecule as an atom-bond-graph (100) comprising atoms of the molecular structure and bonds of the molecular structure as $n_{\text{nodes}}$ nodes of the atom-bond-graph (100), and provides as output the predicted value of the physical and/or chemical property (180a, 180b). The method comprises the steps of extracting (120) for each node of the atom-bond-graph (100) a feature vector of dimension $d_{\text{features}}$, the feature vector comprising a node type, the node type preferably being one of atom, bond and global, and further data on the node in case of the node type being atom or bond, generating a feature matrix of dimension $n_{\text{nodes}} \times$ features based on the extracted $n_{\text{nodes}}$ feature vectors; calculating a squared distance matrix D of dimension $n_{\text{nodes}} \times n_{\text{nodes}}$ based on distances between atoms and bonds of the molecular structure, and applying a trained neural network comprising a transformer using (140) the squared distance matrix D for self-attention decay on the feature matrix to generate a prediction (180a, 180b) of the value of the physical and/or chemical property of the molecule.

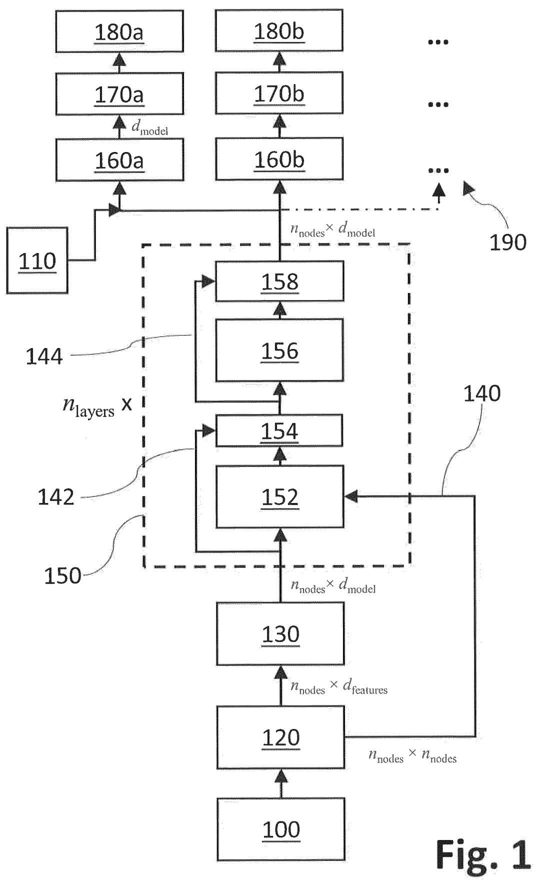


Fig. 1

EP 4 227 951 A1

Processed by Luminess, 75001 PARIS (FR)

## Description

The present technology is related to the field of prediction of physical and/or chemical properties of molecules, especially emitter molecules for the application in OLEDs, and concerns a computer-implemented method for predicting a value of a physical and/or chemical property of a molecule, a computer-implemented method for automated design of a molecule, a computer program having program code for performing one of these methods, a method for training of a neural network for use in these methods, and a system configured to execute these methods and/or the training of such a neural network.

## Introduction

Organic electroluminescent devices containing one or more light-emitting layers based on organics such as, e.g. organic light-emitting diodes (OLEDs), light-emitting electrochemical cells (LECs) and light-emitting transistors gain increasing importance. In particular, OLEDs are promising devices for electronic products such as e.g. screens, displays and illumination devices. In contrast to most electroluminescent devices essentially based on inorganics, organic electroluminescent devices based on organics are often rather flexible and producible in particularly thin layers.

For many applications, including the design of emitter molecules for OLEDs, molecules with optimized physical and/or chemical properties for their respective application are required. However, due to the high number of theoretically feasible molecular structures, it is typically extremely time-consuming to synthesize a sufficient number of possible molecules and measure the desired values of physical and/or chemical properties and/or simulate the molecules and their properties with methods known from prior art and optimize the molecular structures based on these findings. Hence, there is a need for an improved approach for predicting a value of a physical and/or chemical property of a molecule as well as designing a molecule optimized in regard to its physical and/or chemical properties.

## Summary

It is an object of the present invention to provide a method and a system for predicting a value of a physical and/or chemical property of a molecule as well as a method and a system designing a molecule optimized in regard to this physical and/or chemical property. A physical and/or chemical property of a molecule in the meaning of this invention is to be understood as any physical and/or chemical property either of an individual molecule and/or a (molecular) material consisting of a plurality of these molecules. In particular, it is also an object of the present invention to design and optimize a molecule for use as a material for a certain purpose.

This object is achieved by the subject-matter according to the independent claims. Advantageous embodiments are defined in the dependent claims and the following description.

According to a first aspect of the present invention, a computer-implemented method for predicting a value of a physical and/or chemical property of a molecule, especially an emitter molecule and/or a host molecule for use in an OLED, in particular of a TADF (thermally activated delayed fluorescence) emitter, is presented, wherein said method uses as an input a molecular structure of the molecule as an atom-bond-graph, which is a graph comprising atoms and bonds of the molecular structure as $n_{\text{nodes}}$ nodes. Since such a graph can be divided in two disjoint and independent sets (atoms and bonds) connected by edges, it is a bipartite undirected graph. For example, the atom-bond-graph can be generated from a SMILES (simplified molecular input line entry specification) string representing the molecular structure examined. In the context of this invention, a molecular structure of a molecule is to be understood as information on the arrangement of the atoms of the molecule comprising at least the type of bonds between the atoms of the molecule as well as the element of each atom of the molecule.

Optionally, a global node connected to all other nodes can be added when generating the atom-bond-graph. Such a global node can serve as a global attribute to transfer information and/or context that concerns the whole molecule to/from distant nodes for the neural network used in the method. Therefore, it acts as a shortcut for exchanging global information between nodes, bypassing the need for information to travel along edges in multiple steps. Adding a global node often improves prediction performance and therefore is generally highly beneficial.

The method provides as output the predicted value of the physical and/or chemical property (preferably, as a scalar value) and comprises the following steps:

First, for each node of the graph a feature vector of dimension features is extracted, the feature vector comprising a node type (for which one-hot encoding can be used), the node type may for example be "atom", "bond" or "global", and further data on the respective node in case of the node type being atom or bond, called node feature data. The node feature data may comprise for atoms the atomic number (for example as one-hot and/or as integer), a flag for being aromatic,a flag for being part of a ring structure and/or the number of connected implicit hydrogen atoms. For bonds the node feature data may comprise the bond type (i.e., single, double, triple, aromatic).

A feature vector $x$ with dim $x = d_{\text{features}}$ may have the form $x$ = (nodetype, $\text{feat}_{\text{atom}}$, $\text{feat}_{\text{bond}}$), where nodetype

encodes the node type, $feat_{atom}$ the node feature data in case of an atom (atom features), and $feat_{bond}$ the node feature data in case of a bond (bond features). The parameter $node_{type}$ can be a one-hot encoding of the node type, which is one of atom, bond, or global. The use of a global node is optional.

For certain elements of the feature vector it is advantageous to provide the same information in different formats, for example, the atomic number can be added to the feature vector both as integer and as one-hot. One-hot encoding typically makes it easier for the neural network used to treat a specific atomic number in a special way, but removes metric information, i.e., which atomic numbers are similar. For more continuous properties derived from the atomic number, for example the total molecular weight, it is more natural to process the atomic number directly, i.e., as integer.

All in all, $n_{nodes}$ feature vectors are created. From these feature vectors a feature matrix of dimension $n_{nodes} \times d_{features}$ is generated, which may have the form (xi, $x_2$, $x_3$, ..., $x_{n_{nodes}}$), with $x_i$ being feature vectors. Also, a squared distance matrix D of dimension $n_{nodes} \times n_{nodes}$ is calculated based on distances between atoms and bonds of the molecular structure. The squared distance matrix is the $n_{nodes} \times n_{nodes}$ matrix with the ($i$,$j$)-element equal to 0 if $i = j$, and $d_{ij}^2$ if $i \neq j$, where each node is assigned an index and $i$, $j$ are representing such node indices and $d_{ij}$ is the distance between the nodes $i$ and $j$. Accordingly, the squared distance matrix comprises information on distances $d_{ij}$ between atoms and/or bonds of the molecular structure and can be based either on metric distances between nodes (derived from atom coordinates experimentally determined and/or simulated) or on abstract distances between nodes purely derived from the atom-bond-graph without information on atom coordinates.

Finally, the feature matrix is fed into a trained neural network comprising a transformer model (transformer) as machine learning model as first described in Vaswani et al., "Attention Is All You Need", 2017 while using the squared distance matrix D for self-attention decay to generate a prediction of the value of the physical and/or chemical property of the molecule.

Using a method according to the invention, physical and/or chemical properties such as a HOMO (highest occupied molecular orbital) energy level, a LUMO (lowest unoccupied molecular orbital) energy level, a singlet energy level, a triplet energy level, a singlet-triplet energy gap, an oscillator strength, a dipole moment, a photo-luminescent quantum yield, a delayed fluorescence lifetime and/or a peak emission wavelength can be predicted.

Preferably, the method may use a neural network which comprises (as the transformer) a trained transformer-encoder stack with $n_{layers} > 1$, preferably 6, transformer-encoder layers using the squared distance matrix D for self-attention decay, and a trained input encoder (comprising an input encoder layer) configured to generate an input matrix with dimension $n_{nodes} \times d_{model}$ for the transformer-encoder stack generated from the feature matrix of dimension $n_{nodes} \times d_{features}$, where $d_{model}$ is the dimension of the transformer model, for example $d_{model} = 128$. Here, the trained transformer-encoder stack is configured to generate a matrix of dimension $n_{nodes} \times d_{model}$ as output while using the input matrix generated by the input encoder as input. Each of the $n_{layers}$ transformer-encoder layers of the trained transformer-encoder stack after the first layer uses the output of the forgoing layer as input, which is also a matrix of dimension $n_{nodes} \times d_{model}$, as input. Applying the squared distance matrix D has the advantage of reintroducing distance information lost due to the step of generating the feature matrix.

Furthermore, the neural network may comprise a trained projection layer comprising an self-attention layer configured to generate (using the matrix generated by the trained transformer-encoder stack, i.e., its output, as input) a vector of dimension $d_{model}$ as output, and finally, a trained multilayer perceptron (MLP) configured to generate the prediction of the value of the physical and/or chemical property (as target), for example in form of a scalar, from the output vector of the trained projection layer. Preferably, the MLP has one or two hidden layers and/or utilizes ReLU (Rectified Linear Unit) activation and/or dropout. It should be noted that several sets of projection layer plus MLP can be used in parallel, directed at different targets, i.e., a different physical and/or chemical property. In other words, in the final layers of the neural network, the extracted information from the network is combined with an additional self-attention layer (in form of the projection layer) and a feed-forward network (the MLP) per target to predict a set of scalar quantities per molecular structure.

Preferably, the trained transformer-encoder stack consist of $n_{layers}$ transformer-encoder layers with each having a trained multi-head decaying self-attention (i.e., a group of multiple, for example 4, decaying self-attention layers running in parallel), a trained feed-forward network (transformer feed-forward network; for example with a dimension of 512), two trained normalization layers (applying a function LayerNorm and preferably being layer normalizations according to Ba et al., "Layer Normalization", 2016; one for the output of the transformer-encoder layer and one for the output of the feed-forward network), and two residual connections around the transformer-encoder layer and the feed-forward network (sublayers) allowing for an implementation of LayerNorm(x + sublayer(x)), where $x$ is the input of the respective sublayer, as described in Vaswani et al. 2017 and section S7 (Solution 1) of Bratholm et al., "A community-powered search of machine learning strategy space to find NMR property prediction models", 2020. It should be noted that the transformer makes no difference between atoms and bonds. Therefore, and to generate a representations for each atom-bond-graph having identical dimensions, a trained input encoder is used to transform the feature matrix consisting of feature vectors to a matrix of dimension $n_{nodes} \times d_{model}$.

Also preferably, the transformer-encoder layers utilize an attention function for the self-attention decay that is

given by

$$\text{Attention}(Q,K,V) = \text{softmax}\left(\frac{QK^T}{\sqrt{d_k}} - \gamma D\right)V, \quad (1)$$

where $Q$ is a query matrix consisting of query vectors, $K$ a key matrix consisting of key vectors, $V$ a value matrix consisting of value vectors, $d_k$ the dimension of a key vector, $D$ the squared distance matrix, $\gamma$ a trainable parameter, and softmax the softmax function (normalized exponential function) as described in Vaswani et al. 2017 and section S7 (Solution 1) of Bratholm et al., 2020. The matrices $Q$, $K$, $V$ can be calculated using trained matrices from the input matrix of the respective group of self-attention layers of the transformer-encoder stack. In other words, $Q$, $K$, $V$ are calculated from the input matrix of the current self-attention layer, which is the output of the input encoder for the first group of self-attention layers and the output of the previous self-attention layer otherwise, via a trainable linear transformation. Using the squared distance matrix D to modify the matrix within the argument of the softmax function reduces the interaction strength between pairs of faraway nodes of the atom-bond graph. The trainable decay parameter y can be used to scale this influence of the squared distance matrix D and may be initialized as 1. A value of 0 for the parameter y allows attention over the whole graph, whereas for y = ∞ attention is restricted to direct neighbor nodes in the graph. Thus, a distance-scaled self-attention is implemented.

In another advantageous embodiment, the method may use one or more additional input parameters as additional input for the trained multilayer perceptron. These additional input parameters may be physical and/or chemical parameters predicted with a method according to this invention and/or physical and/or chemical parameters predicted using a different method and/or measured physical and/or chemical parameters and/or parameters describing properties of the environment of the molecule under consideration. Such an approach allows for an increased flexibility and is also especially beneficial when training the neural network since typically available experimental or simulated data was generated under different conditions (for example, different emitter concentration and/or host material in case of OLED emitters). Therefore, to be able to use all available data from different experiments for training, the certain conditions of the experiments can be used as additional input features for the multilayer perceptron (i.e., as additional input parameters). After training, the additional input parameters can be set to the desired target conditions.

In a preferred embodiment of the method, the squared distance matrix D is calculated by using distances derived from the atom-bond-graph, i.e., not calculated using physical atom positions. Such a distance between two nodes of the atom-bond-graph is an abstract distance. Preferably, this distance is calculated by the edge distance (number of edges on the shortest path between two nodes) on the atom-bond-graph divided by two and rounded downward. For example, in case of the structure "C1-s4-C2-d5-O3" (s4 being a single, d5 a double bond, the C1, C2 carbon atoms, 03 an oxygen atom, the numbers representing node indices), the distance d between C1 and d5 is $d$(C1,d5) = floor(3/2) = 1.

It was found that a squared distance matrix based on such abstract distances (and not physical distances) is sufficient for most applications, especially when simulating properties of emitter molecules for the use in OLEDs. Beneficially, this approach costs less calculation power than the use of real atom coordinates and requires less computer memory since integers can be used. Therefore, it is possible to simulate physical/chemical properties based on abstract distances extracted from an atom-bond-graph without having 3D information available. This also immensely simplifies the presented method, since no additional simulations are required for estimating atom positions.

In an especially beneficial embodiment of the method, a part of the trained neural network, for example the transformer of the trained neural network, was initialized using transfer learning. It was found that for initialization, parts of another trained neural network suitable for performing one of the methods presented above can be used when having trained this other neural network with suitable data, which might be more easily accessible. In particular, it is possible to pre-train the transformer and the input encoder using values of physical and/or chemical properties as targets, which can be separately be simulated, for example using DFT (density functional theory). This allows for creating a large amount of training data for training of the transformer and the input encoder. Preferably, for training the final model, freshly initialized heads of the neural network, i.e., projection and MLP layers, are first trained with the pre-trained transformer and the input encoder frozen for a defined number of epochs with a constant learning rate. Afterwards, the transformer encoder can be unfrozen and fine-tuned together with the heads with a cosine learning rate schedule.

According to a second aspect of the present invention, a computer-implemented method for predicting a value of a physical and/or chemical property of a molecule can preferably be used for an automated design of a molecule.

In particular, this method for automated design of a molecule, preferably an emitter molecule and/or a host molecule for use in an OLED, in particular of a TADF (thermally activated delayed fluorescence) emitter, uses a genetic algorithm and as inputs a set of starting molecular structures comprising at least one molecular structure, a set of mutation rules for specifying allowed mutations for the genetic algorithm, a scoring function for a molecular structure based on

one or more predicted values of physical and/or chemical properties of the corresponding molecule, and a termination condition. Furthermore, said method provides as output the molecular structure of the designed molecule. It comprises as steps providing the set of starting molecular structures as a population of parent structures to the genetic algorithm, generating a population of offspring molecular structures partially or completely from the population of parent molecular structures using the genetic algorithm by mutating at least one member of the population using the genetic algorithm, and predicting, preferably using a method according to the first aspect of the present invention based on a neural network, one or more values of physical and/or chemical properties of the molecules corresponding to the members of the population of offspring molecular structures and calculating a value for the scoring function for each member of the population of offspring molecular structures based thereon. Here, the one or more values of physical and/or chemical properties are predicted by a neural network using a transformer model with self-attention decay on the molecular structure predicting one or more values of physical and/or chemical properties of the members using the molecular structure as input, wherein the machine-learning model preferably is designed as described further above. Afterwards, the termination condition is checked and in case it is not met, a new population of parent molecular structures consisting of at least one member of the population of offspring molecular structures having the most optimal (might be highest or lowest) value of the scoring function among the offspring molecular structures is generated.

The last steps (generating a population of offspring molecular structures, predicting physical and/or chemical properties of the molecules and calculating values for the scoring function, generating a new population of parent molecular structures) are iterated until the termination condition is met. Afterwards, one of the generated molecular structures is selected as output. This termination condition may be dependent on the value of the scoring function for the most optimal molecular structure in the current generation, but may also or additionally depend on the number of iterations (i.e., only a certain number of iterations are conducted). In particular, the termination condition may be defined as fulfilled if the value of the scoring function for at least one member of the population offspring structure is greater or smaller than or equal to a pre-defined value and/or a pre-defined number of iterations is reached.

In other words, the method optimizes a set of molecules (population) over a set of genetic optimization iterations (generations). In each generation, a set of parent molecules is selected based on a scoring function, for which the value of the scoring function (score) is to be maximized or minimized. The scoring function takes into account a set of physical and/or chemical properties that are estimated with the help of machine learning models. The selected molecules are then modified (mutated) by performing a random set of possible elementary modifications. Finally, for the next generation this process is repeated until the termination condition, for example a certain value of the scoring function is met.

After each generation of new molecular structures, the molecular structures are assigned a scalar value, the score, also called fitness, based on the scoring function. This score describes how well the molecular structures matches the desired target criteria and is optimized by the algorithm by allowing high-scoring molecular structures to survive and produce offspring with a higher probability. For calculating the score, one or more values of physical and/or chemical properties (given as scalar values) are predicted using a neural network with a transformer with self-attention decay on molecular structures, wherein the molecular structures could for example be provided as SMILES strings. It is possible, that a user defines a scoring function (for example a linear ramp, a Gaussian shaped curve or similar) by selecting a set of physical and/or chemical properties (features) that the user is interested in. Each of these features may then be calculated and mapped with a user-configurable scoring function to a feature score in a defined range, for example [0,1], that specifies how well the feature value fits the desired target value. Finally, individual feature scores may be combined, possibly via a user-configurable aggregation function, to a final score per molecular structure.

Preferably, mutating using the genetic algorithm is a fragment-based mutation and/or string-based mutation. For fragment-based mutation, the molecular structures of molecules have to be represented as graphs of interconnected molecule fragments, which are added, removed or replaced for a mutation. In contrast, a string-based mutation is based on string representing a molecular structure and applies elementary (pre-defined) transformations to the string representing the molecular structure at certain positions. For example, a string-based mutation can utilize SMARTS (SMILES arbitrary target specification). In this case, the string is a SMILES (simplified molecular input line entry specification) string and SMARTS pattern matching can be used to find suitable positions for transformations.

In more detail, for fragment-based mutation a molecular structure of a molecule is represented as a graph of interconnected molecule fragments. Here, a molecule fragment (also called fragment) is a representation of a molecular structure with a number of placeholder atoms (symbols used in the graph marking positions where other fragments can be linked to when mutating the fragment) that are connected to exactly one other atom of the fragment and that can be linked to placeholder atoms in different fragments. As placeholder atoms instead of purely abstract symbols also atoms of otherwise not used elements, for example atoms of the actinide series (e.g., Am, Bk), can be used.

In general, a graph representing a molecular structure may be constructed by merging fragments along the linked placeholder atoms by adding a single bond between two atoms connected to the placeholder atoms in the link and removing the placeholder atoms. A graph consisting of fragments is referred to as a fragment graph. Therefore, also a single fragment is a fragment graph. A fragment graph is for example generated by connecting the following three fragments with two Am-Bk links as marked with double arrows:

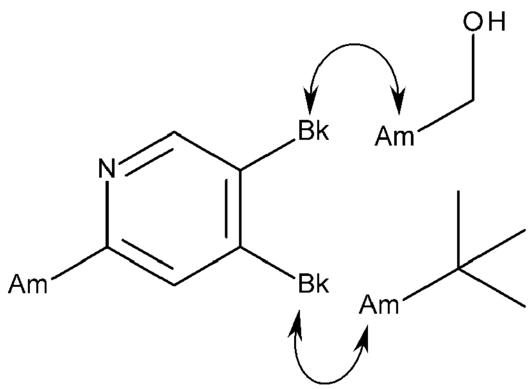

(F1)

After removing the remaining Am atom, the molecule represented is the following:

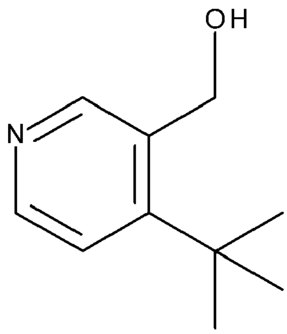

(F2)

Fragment-based mutation can be configured by specifying a set of fragments, for example in the form of a SMILES string, with placeholder atoms as described above and defining for each fragment a positive weight that specifies the relative probability to pick this frequent in a mutation. Additionally, a set of connection rules may be specified for each placeholder atom type, i.e., a list of rules specifying for each placeholder atom type a list of placeholder atom types that this placeholder atom type can be connected to. For example, in case of Am, Bk, and U atoms acting as placeholder atoms in a set of fragments, a connection rule might be that Am atoms can only be connected to Am or Bk atoms and Bk atoms to Am or Bk atoms (which might be denoted as "(Am, Bk)"), but U atoms can only connect to other U atoms. The use of placeholder atoms makes it possible to allow only specific connections between certain fragments and/or sets of fragments.

For mutating a molecular structure using fragment-based mutation several operations are possible. Possible mutations include:

- Addition of leaf fragment: A random fragment with a random free linking position is picked and a random fragment that can be connected to this linking position according to the connection rules is added and linked to the fragment graph. The fragment added is called a leaf fragment, since it is only connected to a single other fragment. For example

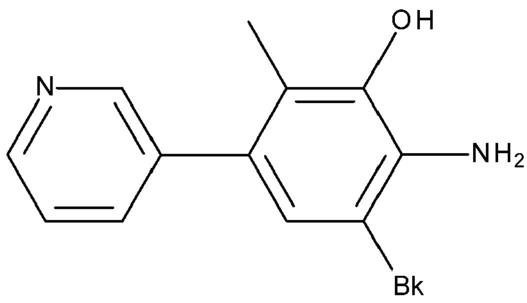

(M1)

is mutated to

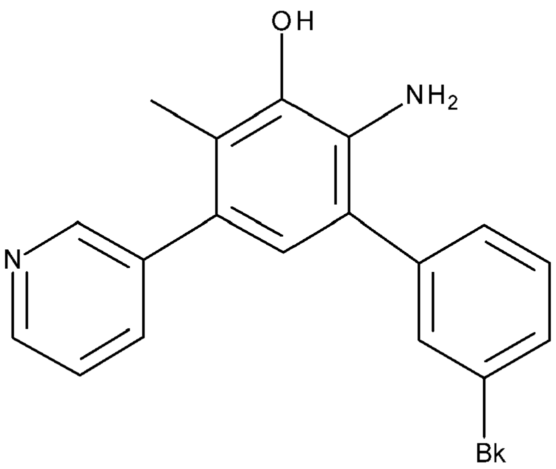

(M2)

- Removal of leaf fragment: A random fragment that is connected to only a single fragment (i.e., a leaf in the fragment graph) is removed from the fragment graph, for example:

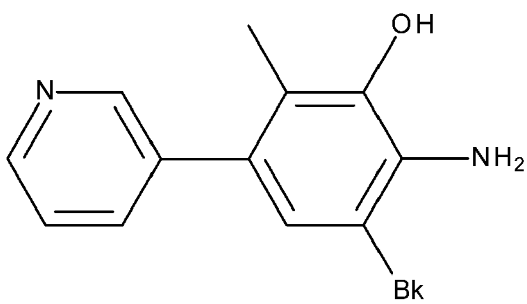

(M3)

is mutated to

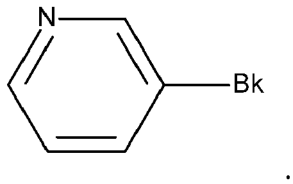

(M4)

- Addition of inner fragment: A random link (connected fragments) is picked and a random fragment that can connect to the corresponding placeholder atoms is inserted at the link position, for example

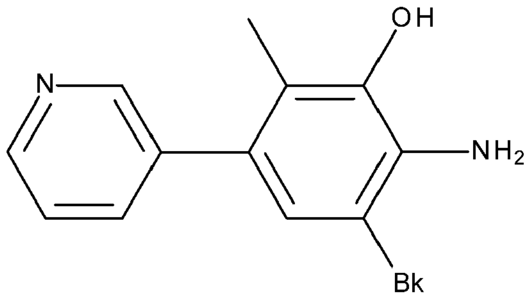

(M5)

is mutated to

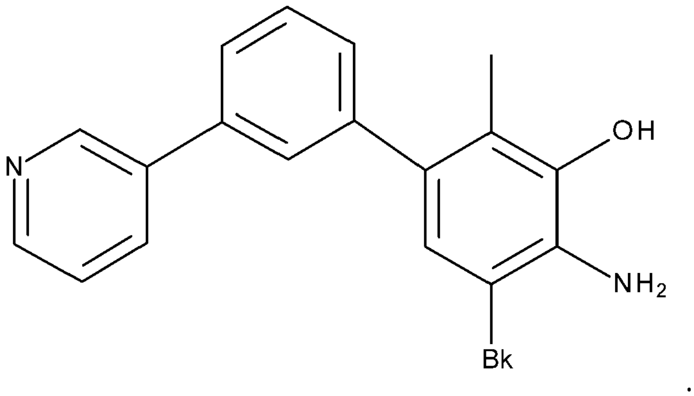

(M6)

- Removal of inner fragment: A random fragment that is connected to two fragments which can be connected according to the connection rules is removed from the fragment graph, for example:

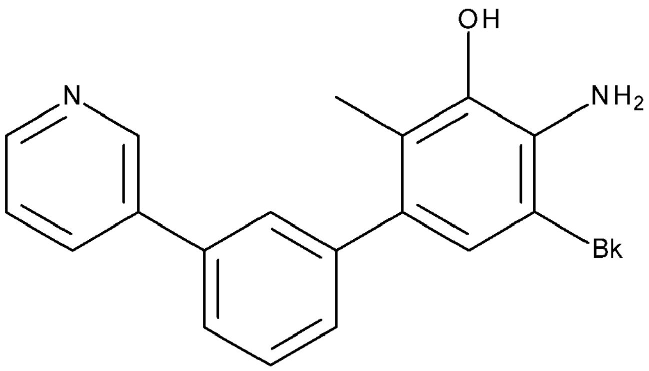

(M7)

is mutated to

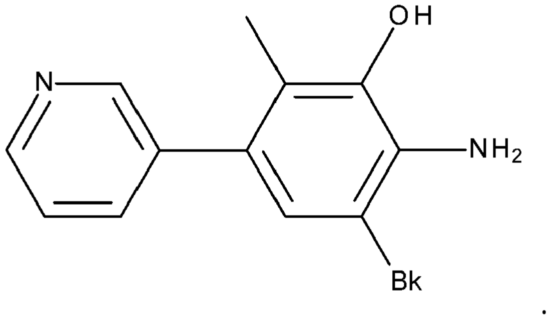

(M8)

- Replacement of fragment: A random fragment is replaced by a random fragment that has at least the amount of placeholder atoms of each type to replace the currently linked fragment (e.g., if the fragment to replace is connected at two Am and one Bk placeholder atoms, a fragment with at least two Am and one Bk placeholder atoms is chosen). The new fragment is linked to the fragment graph at random compatible positions while the current fragment is removed from the fragment graph. For example:

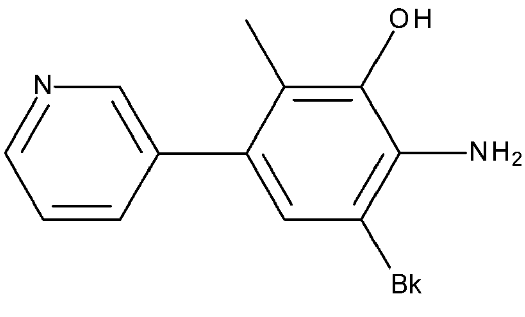

(M9)

is mutated to

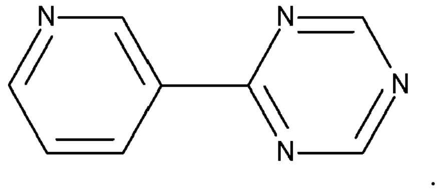

(M10)

- Cross-over: It is also possible that with a defined probability a so-called "cross-over mutation" is performed instead of a normal mutation. A cross-over mutation uses two fragments, i.e., a second individual is sampled from the current pool of parent structures. The two parent molecules (first parent and second parent) are then combined by first splitting each parent molecule at a random link of the same type (e.g. between the same placeholder atom types) and then reconnecting compatible molecule parts from each parent. For example (the child molecule is generated by the encircled molecule parts of the first and second parent):

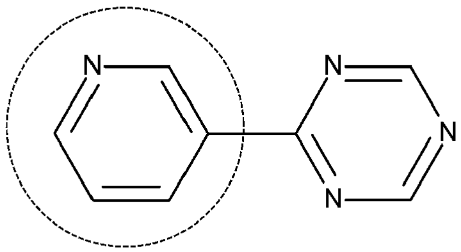

(M11; first parent)

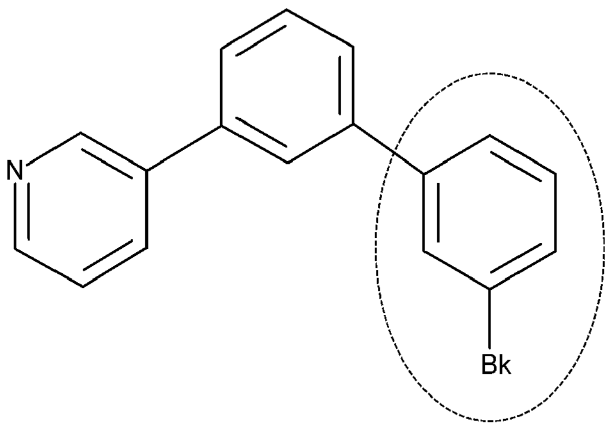

(M12; second parent)

is combined to

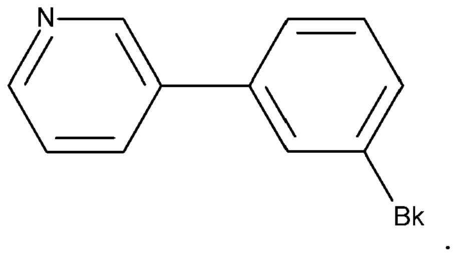

(M13; child)

In all these operations, where a random choice is made, a weighted random choice is also possible instead of an equally distributed one. Typically, when mutating a molecular structure, one of these operations is picked at random or weighted random.

In the following, as an example possible fragments are shown with Am, Bk, and U atoms acting as placeholder atoms (linker atoms):

- Eight donor fragments with Am linker atoms:

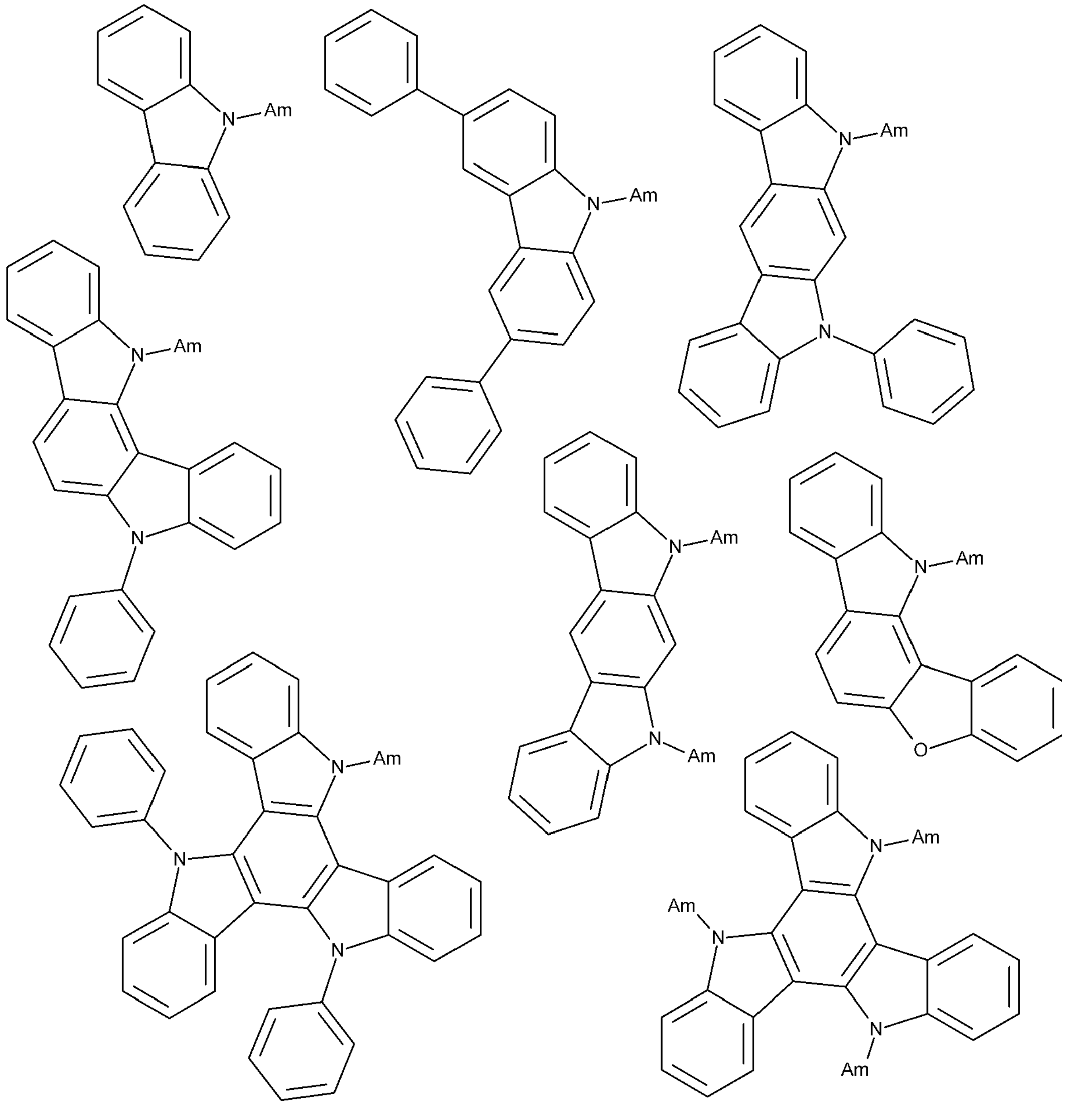

(F3)

- Four acceptor fragments with Bk linker atoms:

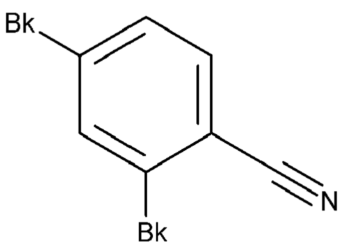

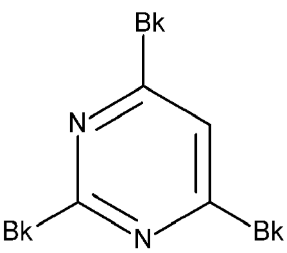

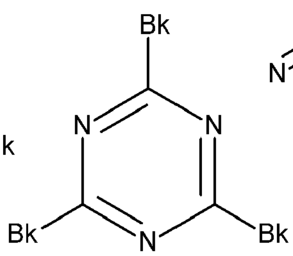

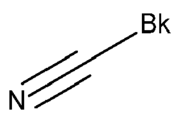

(F4)

- Two spacer fragments with U linker atoms:

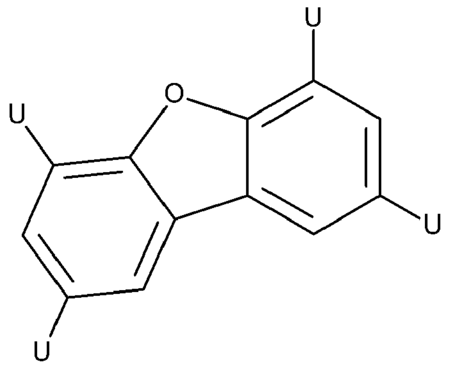

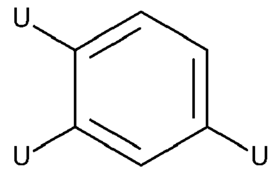

(F5)

These fragments can be used in a method for automated design of a molecule according to the invention using fragment-based mutation and are particularly advantageous for designing of TADF emitters. In this case, connection rules for example can be (Am, Bk), (Am, U), (Bk, U), (U, U), i.e., donors can connect to acceptors or spacers, acceptors can connect to donors or spacers, and spacers can also connect to themselves.

An alternative to a fragment-based mutation is a string-based mutation, which is for example SMARTS-based. Instead of keeping track of a graph of fragments, a string-based mutation strategy represents a molecular structure as a single string, for example as a SMILES string. However, as in the case of the fragment-based mutation, this string may contain additional markers, for example in the form of isotope tags and/or placeholder atoms (also called linker atoms), for example from the actinide series, that are removed for output and scoring. These tags may be used to find specific locations introduced by previous mutations and thereby allow for the definition of fragment-based mutation with the string-based approach as well. Compared with the fragment-based approach, the string-based mutation approach allows for the definition of more fine-grained, elementary molecule manipulations consisting of: addition / removal of bonds, change of atom type / bond type, swapping atom types between neighboring atoms, addition / removal of a substructure at the edge of the molecule or between existing bonds, shifting of substructures connected to one atom to a neighboring atom, substitution of a set of atoms with another set of atoms. All in all, the string-based mutation can be seen as a more general variant of the fragment-based mutation.

Furthermore, it is advantageous for most applications, to use tournament selection and/or elitism for the genetic algorithm. To generate a new generation of parent molecular structures (parent selection) using tournament selection, a certain number of "tournaments" is performed by selecting a defined number of molecular structures of the current population of offspring molecular structures or (in the first iteration) starting molecular structures for each tournament at random. For each tournament, a certain number of the molecular structures with the highest score in the respective tournament are selected to form the set of parent molecular structures for the next generation, i.e., those molecular structures which are used for mutation. Elitism may be used additionally. Here, a defined number of best scoring molecular structures in the current population of offspring molecular structures or (in the first iteration) starting molecular structures is kept without mutation. Instead, they are directly copied to the next generation, i.e., they are automatically included in the next population of offspring molecular structures.

All in all, the best scoring molecular structures (from elitism) and the molecular structures generated (by mutation, for example using tournament selection) form the next population of parent molecular structures.

According to a further aspect of the invention, a system comprising at least one processor and a storage device is configured to execute a computer-implemented method according to one of the methods of the invention. The computer-implemented methods as presented above can be realized as computer programs being executed by such a system.

According to a another aspect of the present invention, a neural network for use in a method according to any of the computer-implemented methods presented above and comprising a transformer configured to use a squared distance matrix D for self-attention decay, one or more projection layers comprising an self-attention layer configured to generate a vector as output using a matrix generated by the transformer as input, and one or more multilayer perceptrons using the output of one of the one or more projection layers as input, can be trained with a method comprising the following steps: First, a data set comprising a molecular structure of a molecule and a value (as ground truth) of a physical and/or chemical property of the molecule is provided, where the physical and/or chemical property is assigned to one

of the multilayer perceptrons. The molecular structure of each molecule is converted to an atom-bond-graph comprising atoms of the molecular structure and bonds of the molecular structure as $n_{\text{nodes}}$ nodes of the atom-bond-graph and for each node of the atom-bond-graph a feature vector of dimension features is extracted, the feature vector comprising a node type, the node type preferably being one of atom, bond and global, and further data (node feature data) on the node in case of the node type being atom or bond. Afterwards, a feature matrix of dimension $n_{\text{nodes}} \times d_{\text{features}}$ based on the extracted $n_{\text{nodes}}$ feature vectors is generated. The squared distance matrix D of dimension $n_{\text{nodes}} \times n_{\text{nodes}}$ based on distances between atoms and bonds of the molecular structure is calculated. Finally, an output value of the multilayer perceptron assigned to the physical and/or chemical property is generated and the neural network adjusted based on comparing the output value of the multilayer perceptron to the value of the physical and/or chemical property assigned to the multilayer perceptron. For comparing the output with the ground truth typically a loss function is used. These steps can be repeated for a certain number of epochs with different data sets comprising a molecular structure of a molecule and a value of a physical and/or chemical property until the neural network has been sufficiently trained.

In an advantageous embodiment of the method the neural network comprises at least two multilayer perceptrons and the steps as described above are repeated for a different data set comprising the molecular structure of the molecule and a value for a different physical and/or chemical property, the different physical and/or chemical property being assigned to a different multilayer perceptron. This embodiment is based on the surprising finding that training for the main parts, especially the transformer, of the neural network as used in the methods according to the first and second aspects of this invention can be performed by using data as ground truth for different physical and/or chemical properties when using multiple multilayer perceptrons as separated outputs of the neural network, i.e., every physical and/or chemical property has to be assigned to a perceptron.

Similarly, a part of the neural network, for example the transformer, can be initialized using the corresponding part of another trained neural network suitable for use in a method according to any the first and second aspect of this invention. As already mentioned, it was found that for initialization, parts of another trained neural network can be used when having trained this other neural network with suitable data, which might be more easily accessible and/or generatable. This way, a large amount of training data can be generated. Preferably, for training the model after initialization using such a pre-trained part of another trained neural network, freshly initialized heads of the neural network including the multiplayer perceptrons are first trained with the pre-trained part for a defined number of epochs with a constant learning rate. Afterwards, the rest of the neural network can be unfrozen and fine-tuned together with the heads with a cosine learning rate schedule. For these training methods, a system comprising at least one processor and a storage device can be used.

## Short Description of the Figures

Hereinafter, specific embodiments are described referring to the drawings, wherein:

Figure 1 shows as a flow chart a possible neural network for use in a method according to the first aspect of the invention,

Figure 2 shows as a flow chart a method according to the second aspect of the invention, and

Figure 3 is a schematic illustration of a system for executing methods for predicting a value of a physical and/or chemical property, for design of a molecule and for training of a neural network according to the invention.

## Detailed Description

Figure 1 shows in a schematic way a possible structure of software program comprising a neural network that may be used in a method according to the present invention. Additionally, this figure also illustrates the underlying method according to the first aspect of the present invention.

An atom-bond-graph 100 describing the molecular structure, for which a value of a physical and/or chemical property should be predicted, is provided (which is, for example, generated from a SMILES string) as input of this software program. This atom-bond-graph 100 is used by a graph feature extractor unit 120, which is a unit of the program which extracts for each node (atom, bond, global) of the atom-bond-graph 100 a feature vector which comprises the node type of the node plus further data on the node in case of an atom or a bond and generates a feature matrix of dimension $n_{\text{nodes}} \times$ features consisting of $n_{\text{nodes}}$ feature vectors with dimension features. Via an input encoder layer 130 this feature matrix is used as an input for a trained input encoder, which generates a matrix with dimension $n_{\text{nodes}} \times d_{\text{model}}$ which is used by an transformer-encoder stack consisting of $n_{\text{layers}}$ transformer-encoder layers 150. Each of these layers 150 comprises a trained multi-head decaying self-attention 152, a trained transformer feed-forward neural network 156, two layer normalizations 154, 158 and two residual connections 142, 144, which connect the input of the transformer-encoder

layer with the first layer normalization 154 and the input of the feed-forward neural network 156 with the second layer normalization 158.

The multi-head decaying self-attention 152 consists of multiple parallel decaying self-attention layers, for example 4. The decaying self-attention can be implemented by using a function of the form as shown in formula (1).

The required matrix D used (arrow 140) is a squared distance matrix of dimension $n_{\text{nodes}} \times n_{\text{nodes}}$ calculated from the atom-bond-graph and may for example also be calculated and provided by the feature extractor unit 120 of the software.

The transformer encoder stack generates a matrix of dimensions $n_{\text{nodes}} \times d_{\text{model}}$ that is projected by one or more trained projection layers 160a, 160b, one for each target, comprising an self-attention layer to vectors of dimension $d_{\text{model}}$. In the figure, two projection layers 160a, 160b are shown, but more (190) are possible, dependent on the number of targets 180a, 180b. These vectors are used by MLPs 170a, 170b, which based on these vectors and optionally on one or more additional input parameters 110 (for example a property of a host molecule, a concentration, a solvent) finally calculate the targets 180a, 180b, i.e., the values of the physical and/or chemical properties as scalar values. As is the case for projection layers, the number of MLPs 170a, 170b depends on the number of targets 180a, 180b and can be more (190) than the two shown in the Figure.

Figure 2 shows as a flow chart for use in a method according to the second aspect of the invention. After initialization 200 of the genetic algorithm, which comprises providing at least one molecular structure as stating molecular structure and the other required parameters (mutation rules, scoring function, termination condition), for example in form of a configuration file, in the parent selection step 220 the starting molecular structures are defined as parent molecular structures. Afterwards, in step 240 a fragment-based or string-based mutation is applied to produce a population of offspring molecular structures. In step 260 at least for these molecular structures at least one value of a physical and/or chemical property is predicted as a scalar value using the method according to the first aspect of the present invention and a value for a scoring function is calculated using this predicted value of the physical and/or chemical property. Afterwards the termination condition is checked in step 270, which might be dependent on the calculated value of the scoring function.

If the termination condition is not fulfilled (arrow 210), a new set of parent molecular structures are generated in step 220. For this, for example tournament selection can be used. The steps of generating a new parent generation (parent molecular structures) 220, generating a population of offspring molecular structures (using mutation) 240, predicting physical and/or chemical properties of the molecules and determining the score 260, and checking the termination condition 270 (these steps forming one iteration 290 of the process) are repeated until the termination condition is met. In case the termination condition is fulfilled, in step 280 one or more of the generated molecular structures are provided, which are typically the highest-scoring molecular structures generated.

Figure 3 shows a system 300 for executing methods for predicting a value of a physical and/or chemical property and/or for designing a molecule and/or for training of a neural network according to the invention. The system 300 comprises at least one processor 340 connected to at least one storage device 350 and may comprise or may be a computer device, for example a personal computer or a mobile device. The system 300 or parts of the system may also be a distributed computing system, for example a cloud computing system.

A processor 340 of the system 300 may be for example a microprocessor, a central processing unit (CPU), an application-specific integrated circuit (ASIC), a graphics processing unit (GPU), a tensor processing unit (TPU) or a field programmable gate array (FPGA). A storage device 350 used may be for example a random access memory (RAM), a hard drive (HD), a solid-state drive (SSD), a flash memory, a compact disk (CD) or a digital video disk (DVD). The system may also comprise one or more display devices and one or more controllers, for example a keyboard, a computer mouse and/or a touch screen.

The system 300 may be configured to execute a method for predicting a value of a physical and/or chemical property according to the invention and in this case may receive as input 310, for example provided by a human operator or a data bank 380, a molecular structure of the molecule in computer-readable form, for example as a SMILES string. This input 310 is converted by the system 300 to an atom-bond-graph 100 comprising atoms of the molecular structure and bonds of the molecular structure as $n_{\text{nodes}}$ nodes of the atom-bond-graph 100. The system 300 may also be configured to receive 330 such an atom-bond-graph 100 as input 310 directly. Furthermore, the system 300 is also preferably configured to provide as output 320 the predicted value of the physical and/or chemical property after executing the method for predicting a value of the physical and/or chemical property according to the invention.

Alternatively or additionally, the system 300 may also be configured to execute a method for automated design of a molecule according to the invention and in this case may receive as input 300, for example provided by a human operator or a data bank 380, a set of starting molecular structures, for example as a SMILES string, comprising at least one molecular structure, a set of mutation rules for specifying allowed mutations for a genetic algorithm, a scoring function for a molecular structure based on one or more predicted values of the physical and/or chemical properties of the corresponding molecule, and a termination condition. The system 300 may also be configured to provide as output 320 the molecular structure of the by the method designed molecule.

Alternatively or additionally, the system 300 may also be configured for executing a method for training of a neural network for use in a method according to the invention. In this case, the system 300 is designed to receive input training data, for example provided by a human operator or a data bank 380, comprising molecular structures of different molecules and values of physical and/or chemical properties of the molecules as input 310.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

## Claims

1. A computer-implemented method for predicting a value of a physical and/or chemical property (180a, 180b) of a molecule, said method using as input a molecular structure of the molecule as an atom-bond-graph (100) comprising atoms of the molecular structure and bonds of the molecular structure as $n_{nodes}$ nodes of the atom-bond-graph (100), said method providing as output the predicted value of the physical and/or chemical property (180a, 180b), comprising the following steps:

   a) Extracting (120) for each node of the atom-bond-graph (100) a feature vector of dimension $d_{features}$, the feature vector comprising a node type, the node type preferably being one of atom, bond and global, and further data on the node in case of the node type being atom or bond;
   b) Generating a feature matrix of dimension $n_{nodes}$ × features based on the extracted $n_{nodes}$ feature vectors;
   c) Calculating a squared distance matrix D of dimension $n_{nodes} \times n_{nodes}$ based on distances between atoms and bonds of the molecular structure;
   d) Applying a trained neural network comprising a transformer using (140) the squared distance matrix D for self-attention decay on the feature matrix to generate a prediction (180a, 180b) of the value of the physical and/or chemical property of the molecule.

2. The computer-implemented method according to claim 1, **characterized in that** the neural network comprises:

   • a trained input encoder (130) configured to generate an input matrix with dimension $n_{nodes} \times d_{model}$,
   • as the transformer a trained transformer-encoder stack with $n_{layers} > 1$ transformer-encoder layers (150) using the squared distance matrix D for self-attention decay, wherein the trained transformer-encoder stack is configured to generate a matrix of dimension $n_{nodes} \times d_{model}$ as output using the input matrix of the input encoder (130) as input,
   • a trained projection layer (160a, 160b) comprising a self-attention layer configured to generate a vector of dimension $d_{model}$ as output using the matrix generated by the trained transformer-encoder stack as input,
   • a trained multilayer perceptron (170a, 170b) configured to generate the prediction of the value of the physical and/or chemical property from the output vector of the trained projection layer.

3. The computer-implemented method according to claim 2, **characterized in that** each of the $n_{layers}$ trained transformer-encoder layers comprises a trained multi-head decaying self-attention (152), a trained feed-forward network (156), two trained layer normalizations (154, 158) and two residual connections (142, 144).

4. The computer-implemented method according claim 2 or 3, **characterized in that** an additional input parameter (110) is used as an additional input for the trained multilayer perceptron (170a, 170b).

5. The computer-implemented method according to any one of the preceding claims, **characterized in that** the physical and/or chemical property is one of a HOMO energy level, a LUMO energy level, a singlet energy level, a triplet energy level, a singlet-triplet energy gap, an oscillator strength, a dipole moment, a photo-luminescent quantum yield, a delayed fluorescence lifetime and/or a peak emission wavelength.

6. The computer-implemented method according to any one of the preceding claims, **characterized in that** the squared distance matrix D is calculated by using abstract distances derived from the atom-bond-graph.

7. The computer-implemented method according to any one of the preceding claims **characterized in that** a part of

the trained neural network, for example the transformer of the trained neural network, was initialized by another transformer of another trained neural network suitable for performing a method according to any one of the preceding claims.

**8.** A computer-implemented method for automated design of a molecule, preferably of an emitter molecule for use in an OLED, in particular of a TADF emitter, said method using a genetic algorithm and as inputs (200) a set of starting molecular structures comprising at least one molecular structure,

a set of mutation rules for specifying allowed mutations for the genetic algorithm,
a scoring function for a molecular structure based on one or more predicted values of the physical and/or chemical properties of the corresponding molecule, and
a termination condition,
said method providing as output the molecular structure of the designed molecule, comprising the following steps:

a) Providing (220) the set of starting molecular structures as a population of parent structures to the genetic algorithm;
b) Generating (240) a population of offspring molecular structures partially or completely from the population of parent structures using the genetic algorithm by mutating at least one member of the population using the genetic algorithm;
c) Predicting (260), preferably using a method according to any one of the claims 1 to 7, one or more values of the physical and/or chemical properties of the molecules corresponding to the members of the population of offspring molecular structures and calculating a value for the scoring function for each member of the population of offspring molecular structures based thereon,
wherein the one or more values of the physical and/or chemical properties are predicted using a neural network using a transformer with self-attention decay on molecular structures predicting one or more values of the physical and/or chemical properties of the members using the molecular structure as input;
d) Checking (270) if the termination condition is met and if not (210), generating (220) a new population of parent molecular structures consisting of at least one member of the population of offspring molecular structures having the most optimal value of the scoring function among the offspring molecular structures;
e) Iterating (210) steps b, c, and d until the termination condition is met;
f) Selecting (280) a molecular structure generated in step b or any of its iterations (290) as an output.

**9.** The computer-implemented method according to claim 8, **characterized in that** the termination condition is met if the scoring function for at least one member of the population offspring structure is greater or smaller than or equal to a pre-defined value and/or a pre-defined number of iterations (290) of steps b, c, d is reached.

**10.** The computer-implemented method according to claim 8 or 9, **characterized in that** the mutating using the genetic algorithm is a fragment-based mutation and/or string-based mutation.

**11.** The computer-implemented method according to any one of claims 8 to 10, **characterized in that** the genetic algorithm uses tournament selection and/or elitism.

**12.** A method for training of a neural network for use in a method according to any one of claims 1 to 11, wherein the neural network comprises a transformer configured to use a squared distance matrix D for attention decay, one or more projection layers comprising an attention layer configured to generate a vector as output using a matrix generated by the transformer as input, and one or more multilayer perceptrons (170a, 170b) using the output of one of the one or more projection layers as input,
the method comprising the following steps:

a) Providing a data set comprising a molecular structure of a molecule and a value of a physical and/or chemical property of the molecule, where the physical and/or chemical property is assigned to one of the multilayer perceptrons;
b) Converting the molecular structure of each molecule as an atom-bond-graph (100) comprising atoms of the molecular structure and bonds of the molecular structure as $n_{\text{nodes}}$ nodes of the atom-bond-graph (100);
c) Extracting for each node of the atom-bond-graph (100) a feature vector of dimension $d_{\text{features}}$, the feature vector comprising a node type, the node type preferably being one of atom, bond and global, and further data on the node in case of the node type being atom or bond;
d) Generating a feature matrix of dimension $n_{\text{nodes}} \times d_{\text{features}}$ based on the extracted $n_{\text{nodes}}$ feature vectors;

e) Calculating the squared distance matrix D of dimension $n_{\text{nodes}} \times n_{\text{nodes}}$ based on distances between atoms and bonds of the molecular structure;
f) Generating an output value (180a, 180b) of the multilayer perceptron (170a, 170b) assigned to the physical and/or chemical property; and
g) Adjusting the neural network based on comparing the output value of the multilayer perceptron to the value of the physical and/or chemical property assigned to the multilayer perceptron.

**13.** The method according to claim 12, wherein the neural network comprises at least two multilayer perceptrons (170a, 170b), **characterized in that** the steps a to g are repeated for a different data set comprising the molecular structure of the molecule and a value of a different physical and/or chemical property, the different physical and/or chemical property being assigned to a different multilayer perceptron.

**14.** The method according to claim 12 or 13, **characterized in that** the steps a to g are repeated for a different data set comprising the molecular structure of a different molecule and a value of the physical and/or chemical property of the different molecule.

**15.** The method according to any one of the claims 12 to 14, **characterized in that** a part of the neural network, for example the transformer, is initialized using another transformer of another trained neural network suitable for use in a method according to any one of the claims 1 to 11.

**16.** A system (300) comprising a processor (340) and a storage device (350),
wherein the system (300) is configured to execute a computer-implemented method according to any one of the claims 1 to 11 and/or a method for training of a neural network according to any one of claims 12 to 15.

**17.** A computer program having a program code for performing a method according to any one of the claims 1 to 11 when the computer program is executed by a system (300) according to claim 16.

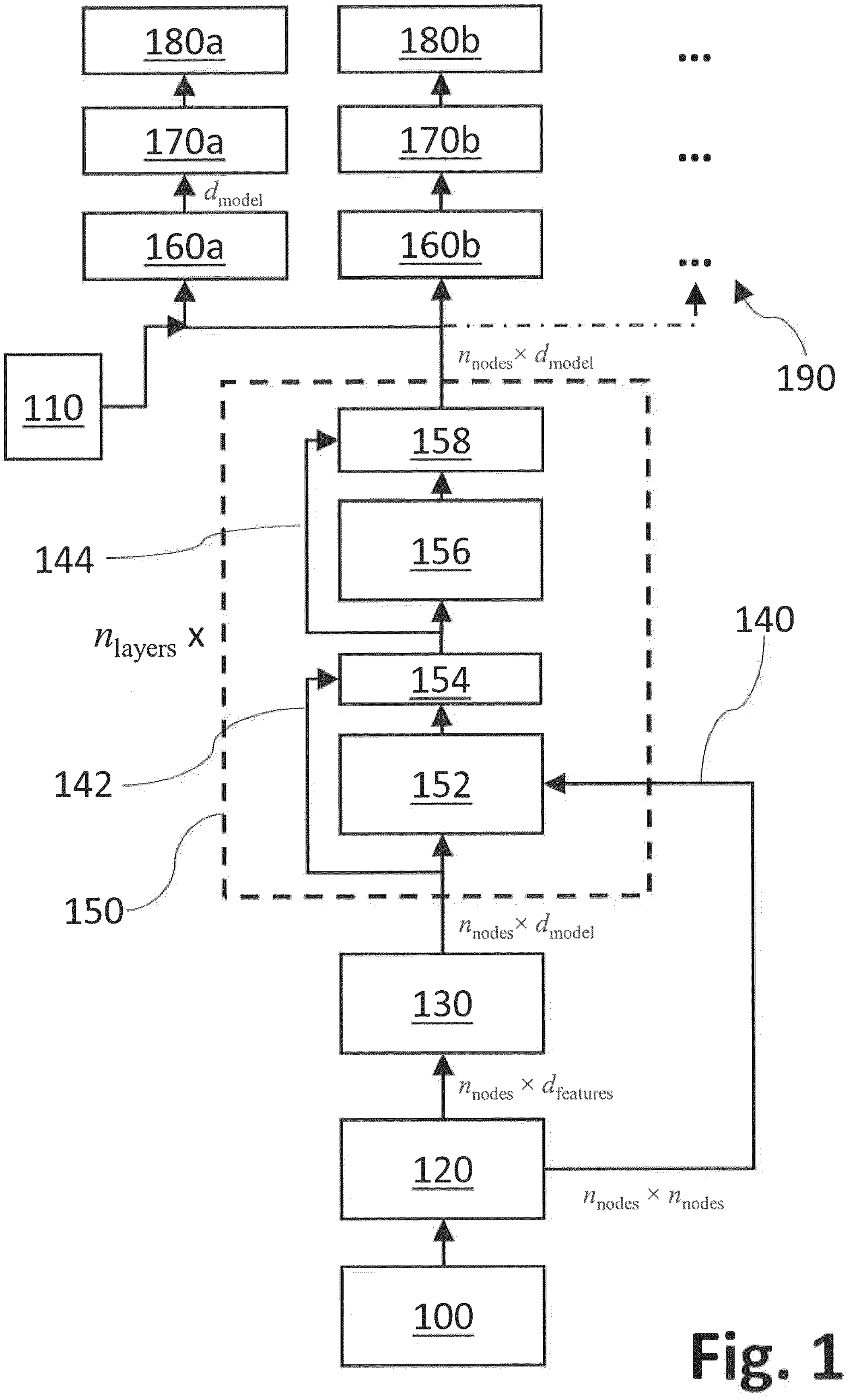
180a
180b
...
170a
170b
...
$d_{model}$
160a
160b
...
110
$n_{nodes} \times d_{model}$
190
158
144
156
$n_{layers}$ x
140
154
142
152
150
$n_{nodes} \times d_{model}$
130
$n_{nodes} \times d_{features}$
120
$n_{nodes} \times n_{nodes}$
100

Fig. 1

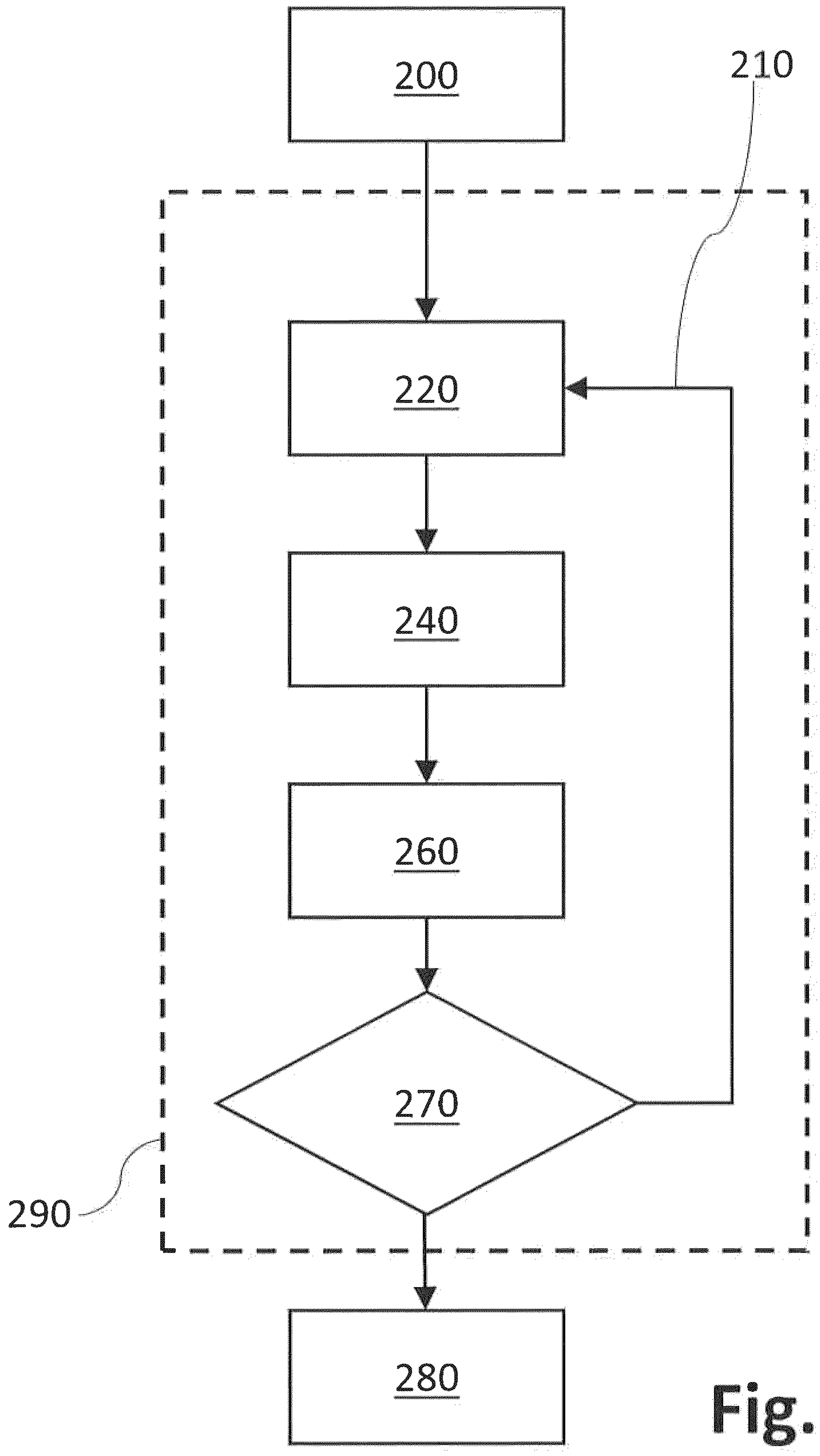


Fig. 2

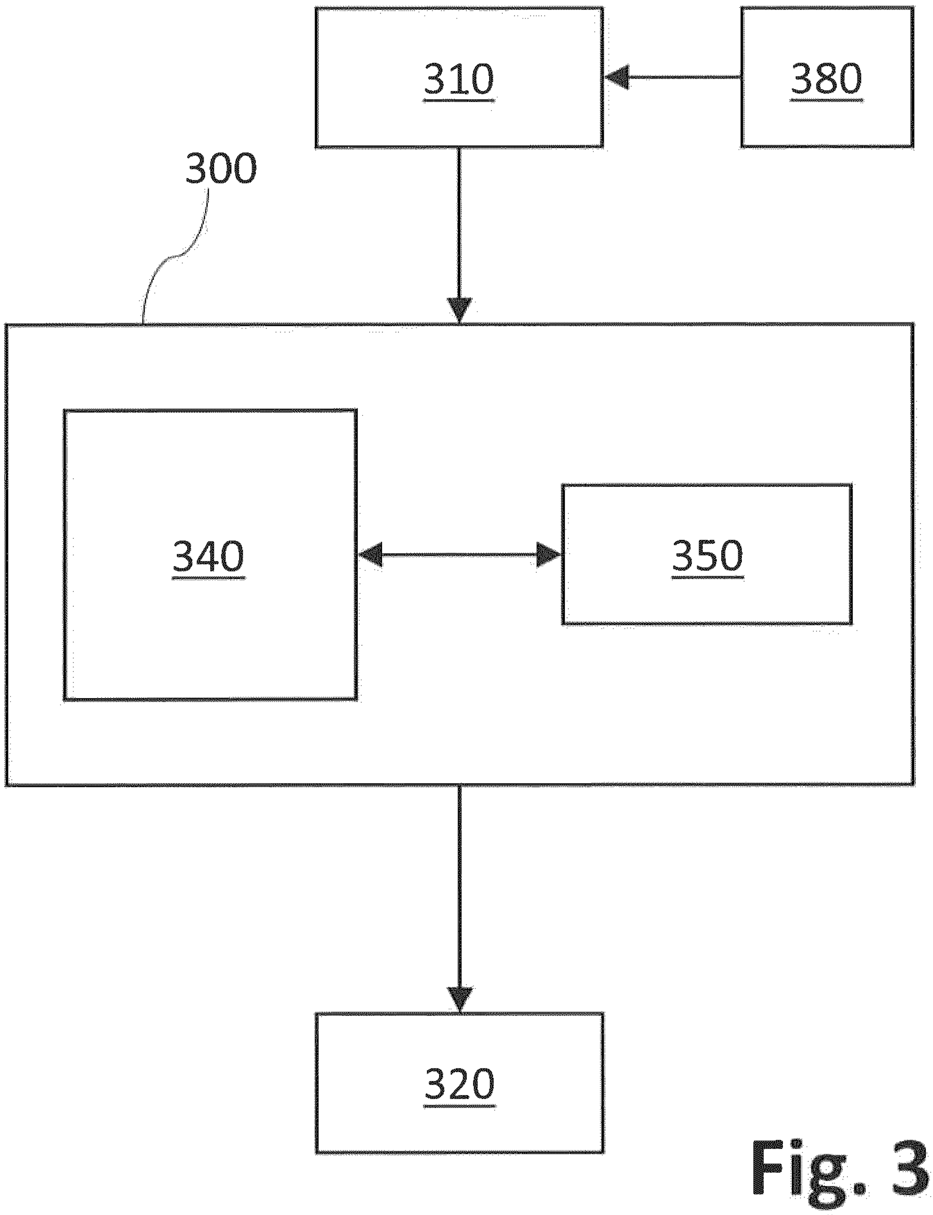


Fig. 3

Europäisches Patentamt
European Patent Office
Office européen des brevets

# EUROPEAN SEARCH REPORT

Application Number
EP 22 15 6423

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LUKASZ MAZIARKA ET AL: "Molecule Attention Transformer", ARXIV.ORG, CORNELL UNIVERSITY LIBRARY, 201 OLIN LIBRARY CORNELL UNIVERSITY ITHACA, NY 14853, 19 February 2020 (2020-02-19), XP081876709, | 12-17 | INV. G16C20/30 G16C20/50 G16C20/70 |
| A | * the whole document * | 1-11 | |
| X | Kwak Bumju ET AL: "Geometry-aware Transformer for molecular property prediction", , 29 June 2021 (2021-06-29), XP055944615, Retrieved from the Internet: URL:https://arxiv.org/pdf/2106.15516.pdf [retrieved on 2022-07-20] | 12-17 | |
| A | * the whole document * | 1-11 | |
| X | YONI CHOUKROUN ET AL: "Geometric Transformer for End-to-End Molecule Properties Prediction", ARXIV.ORG, CORNELL UNIVERSITY LIBRARY, 201 OLIN LIBRARY CORNELL UNIVERSITY ITHACA, NY 14853, 26 October 2021 (2021-10-26), XP091083679, | 12-17 | TECHNICAL FIELDS SEARCHED (IPC): G16C G06N |
| A | * the whole document * | 1-11 | |

-/--

The present search report has been drawn up for all claims

2

| Place of search | Date of completion of the search | Examiner |
|---|---|---|
| The Hague | 14 October 2022 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office / Europäisches Patentamt / Office européen des brevets

# EUROPEAN SEARCH REPORT

Application Number
EP 22 15 6423

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br><br><br>A | QIAN CHEN ET AL: "Directed graph attention neural network utilizing 3D coordinates for molecular property prediction", COMPUTATIONAL MATERIALS SCIENCE, ELSEVIER, AMSTERDAM, NL, vol. 200, 5 August 2021 (2021-08-05), XP086811206, ISSN: 0927-0256, DOI: 10.1016/J.COMMATSCI.2021.110761 [retrieved on 2021-08-05]<br>* the whole document *<br>----- | 12-17<br><br><br><br><br><br><br><br><br><br><br>1-11 | |
| X<br><br><br><br><br><br><br><br>A | LARS A BRATHOLM ET AL: "A community-powered search of machine learning strategy space to find NMR property prediction models", ARXIV.ORG, CORNELL UNIVERSITY LIBRARY, 201 OLIN LIBRARY CORNELL UNIVERSITY ITHACA, NY 14853, 13 August 2020 (2020-08-13), XP081740165,<br>* the whole document *<br>----- | 12-17<br><br><br><br><br><br><br><br>1-11 | |
| X<br><br><br><br><br><br>A | LUKASZ MAZIARKA ET AL: "Relative Molecule Self-Attention Transformer", ARXIV.ORG, CORNELL UNIVERSITY LIBRARY, 201 OLIN LIBRARY CORNELL UNIVERSITY ITHACA, NY 14853, 12 October 2021 (2021-10-12), XP091075933,<br>* the whole document *<br>-----<br>-/-- | 12-17<br><br><br><br><br><br>1-11 | TECHNICAL FIELDS SEARCHED (IPC) |

The present search report has been drawn up for all claims

| Place of search | Date of completion of the search | Examiner |
|---|---|---|
| The Hague | 14 October 2022 | Denoual, Matthieu |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 2 of 3

Europäisches Patentamt
European Patent Office
Office européen des brevets

# EUROPEAN SEARCH REPORT

Application Number
EP 22 15 6423

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YONI CHOUKROUN ET AL: "Geometric Transformer for End-to-End Molecule Properties Prediction", ARXIV.ORG, CORNELL UNIVERSITY LIBRARY, 201 OLIN LIBRARY CORNELL UNIVERSITY ITHACA, NY 14853, 16 December 2021 (2021-12-16), XP091110757, | 12-17 | |
| A | * the whole document * ----- | 1-11 | |
| X | NATHAN BROWN ET AL: "GuacaMol: Benchmarking Models for de Novo Molecular Design", JOURNAL OF CHEMICAL INFORMATION AND MODELING, vol. 59, no. 3, 19 March 2019 (2019-03-19), pages 1096-1108, XP055731734, US ISSN: 1549-9596, DOI: 10.1021/acs.jcim.8b00839 * Sections 1, 2, 7.4.3 and 7.4.4 * ----- | 8-11 | TECHNICAL FIELDS SEARCHED (IPC) |
| X | US 2005/240355 A1 (BROWN NATHAN [NL] ET AL) 27 October 2005 (2005-10-27) * the whole document * ----- | 8-11 | |
| X | DAVID HECHT ET AL: "A Novel In Silico Approach to Drug Discovery via Computational Intelligence", JOURNAL OF CHEMICAL INFORMATION AND MODELING, vol. 49, no. 4, 27 April 2009 (2009-04-27), pages 1105-1121, XP055091733, ISSN: 1549-9596, DOI: 10.1021/ci9000647 * the whole document * ----- | 8-11 | |

The present search report has been drawn up for all claims

2

| Place of search | Date of completion of the search | Examiner |
|---|---|---|
| The Hague | 14 October 2022 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

Europäisches Patentamt
European Patent Office
Office européen des brevets

Application Number

EP 22 15 6423

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [X] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number

EP 22 15 6423

The Search Division considers that the present European patent application does not comply with the requirements of unity of invention and relates to several inventions or groups of inventions, namely:

**1. claims: 1-7, 12-15(completely); 16, 17(partially)**

**Method of predicting a property of a molecule**

---

**2. claims: 8-11(completely); 16, 17(partially)**

**Method of designing molecules**

---

## ANNEX TO THE EUROPEAN SEARCH REPORT ON EUROPEAN PATENT APPLICATION NO. EP 22 15 6423

This annex lists the patent family members relating to the patent documents cited in the above-mentioned European search report. The members are as contained in the European Patent Office EDP file on
The European Patent Office is in no way liable for these particulars which are merely given for the purpose of information.

14-10-2022

| Patent document cited in search report | Publication date | Patent family member(s) | Publication date |
|---|---|---|---|
| US 2005240355 A1 | 27-10-2005 | EP 1589463 A1 | 26-10-2005 |
| | | US 2005240355 A1 | 27-10-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **VASWANI et al.** *Attention Is All You Need,* 2017
- **BA et al.** *Layer Normalization,* 2016
- **BRATHOLM et al.** *A community-powered search of machine learning strategy space to find NMR property prediction models,* 2020